# EUROPEAN PATENT SPECIFICATION

(11) **EP 3 350 169 B1**
(45) Date of publication and mention of the grant of the patent: **16.10.2019**
(21) Application number: 16784598.1
(22) Date of filing: 27.07.2016
(51) Int. Cl.: C07D 233/16

(54) **METHOD FOR SYNTHESIZING A CORROSION INHIBITOR**
VERFAHREN ZUR SYNTHESE EINES KORROSIONSINHIBITORS
PROCÉDÉ DE SYNTHÈSE D'UN INHIBITEUR DE CORROSION

(30) Priority: 18.09.2015 TR 201511715
(43) Date of publication of application: 25.07.2018
(73) Proprietor: Turkiye Petrol Rafinerileri A.S. Tupras, 41780 Korfez/Kocaeli (TR)
(72) Inventor: GUMRUK, Esra, 34775 Umraniye/Istanbul (TR); SECKIN, Oznur Cansu, Bahcesehir/Istanbul (TR); TEKE, Yesim, Sisli/Istanbul (TR)
(74) Representative: Cayli, Hülya
(86) International application number: PCT/TR2016/000101
(87) International publication number: WO 2017/048204

(56) References cited:
- WO-A1-2012/061094
- CN-A- 103 602 328
- GB-A- 1 317 741
- US-A1- 2006 013 798

## Description

### Field of the Invention

The present invention relates to a method for synthesizing a corrosion inhibitor from waste materials so as to be used in petroleum refinery processes.

### Background Art

In petroleum refineries, pipelines are used to transport fluids from a location to another. Said pipelines are mainly made of metal materials such as iron/steel. Although physical resistance of the pipelines made of metal materials is high, chemical structure of the pipeline may deteriorate due to the wear effect of the fluid transported in the pipeline. During the transportation of a salt and/or acid containing fluid in the pipeline, corrosion is experienced in the pipeline because of the wear effect of the fluid. Corrosion is also called as rusting experienced in the materials as a result of chemical and electrochemical reactions, with the effect of the medium where metal or metal alloys are contained. In the pipelines, holes occur over time due to corrosion, which makes the pipeline unusable.

In particular, salts contained in crude oil result in corrosion in the pipelines where crude oil is transported and in equipments of the petroleum refineries such as condenser, heat exchanger, column overhead tray and reflux drum, thereby reducing the lifetime of these structures. In order to prevent crude oil from damaging the refinery equipments, desalters are used in the prior art which remove salts contained in the crude oil therefrom. As disclosed in US2014251874A1, said desalters remove salts in the crude oil by means of a filtration method. However, in such applications, not all of the salts in the crude oil are removed from the oil. Therefore, inhibitors are used in the prior art which reduce corrosion effect of the salts in the crude oil. Said corrosion inhibitors are classified as organic or inorganic inhibitors based on their chemical features: and as anodic, cathodic or both anodic and cathodic inhibitors that form film on the surface, based on their inhibition mechanism. Organic inhibitors take the forefront in that they inhibit both anodic and cathodic reactions on the surface and form film thereon, and they have environment-friendly features. Organic corrosion inhibitors have a high inhibitory effect in that they contain heteroatoms such as oxygen, nitrogen and sulphur and they form primary active centers for adsorption with their ring structure and branched long chains. The functional groups of polymers used as corrosion inhibitors react with metal ions and metal surfaces, thereby forming a film layer on the surface. Polymer-containing inhibitors disperse on the surface due to their heteroatom content and long chain structure and form a protective active surface over a wide area, whereby corrosive effect in the medium is reduced. It is a major advantage to use polymeric materials as inhibitors since they do not contain toxic components and they are environment-friendly and low-cost.

CN 103 602 328 A discloses a method for synthesizing a quaternized imidazoline-based corrosion inhibitor.

GB 1 317 741 A discloses a imidazoline-based corrosion inhibitor and its preparation from a mixture of melted fatty acids, which were obtained as waste products in the production of margarine, and ethylenediamine or polyethylene polyamine in the presence of hexamethylenetetramine

### Brief Description of the Invention

With the present invention, there is provided a method for synthesizing a corrosion inhibitor using waste materials. In the invention, waste vegetable oils are used as raw materials so as to obtain corrosion inhibitors. To this end, the method of synthesizing a corrosion inhibitor using waste vegetable oils comprises the steps of heating the waste vegetable oils to a predetermined first temperature value in order to melt fatty acids contained in the oil; after said fatty acids are melted, adding diethylenetriamine into the fatty acids in a weight ratio of 1/1 to 1/2 of the fatty acid to diethylenetriamine; heating the mixture to a predetermined second temperature value in order to perform esterification reactions; heating the fatty acids esterified through the esterification reactions to a predetermined third temperature value and keeping it for a predetermined first time period in order to obtain an imidazoline ring structure; cooling the resulting imidazoline ring structure to a predetermined fourth temperature value and adding chloroacetic acid thereto and keeping it for a predetermined second time period to perform a quaternarization reaction in order to obtain an imidazoline-based corrosion inhibitor, wherein the esterification reaction comprises the step of adding at least one catalyst which is ethyl acetate into the mixture of fatty acid and diethylenetriamine.

In the method of synthesizing a corrosion inhibitor according to the present invention, since waste vegetable oils are used as raw material, production costs are kept low. Furthermore, given that said waste materials are harmful to the environment, utilization of such materials in synthesis of a corrosion inhibitor contributes to the protection of the environment.

### Object of the Invention

An object of the present invention is to provide a method for synthesizing a corrosion inhibitor which is particularly intended to be used in petroleum refineries.

Another object of the present invention is to provide a method for synthesizing a corrosion inhibitor using waste materials.

Yet another object of the present invention is to provide an easy, reliable and inexpensive method for synthesizing a corrosion inhibitor.

### Description of the Invention

In petroleum refineries, especially due to the wear effect of the salts contained in the crude oil, corrosion may occur in the pipelines and in other equipments of the petroleum refineries (i.e. condenser, heat exchanger, column overhead tray and reflux drum of the refinery). Corrosion in the said equipments results in non-utilization of the equipment. In the prior art, corrosion inhibitors are used to prevent said corrosion. With the present invention, there is provided a method for easy and economical synthesis of the corrosion inhibitors.

In the method for synthesizing a corrosion inhibitor according to the present invention, waste vegetable oils are used as raw materials. In the invention, the method for synthesizing a corrosion inhibitor using waste vegetable oils comprises the steps of heating the waste vegetable oils to a predetermined first temperature value (i.e. 50-80°C) in order to melt fatty acids contained in the oil (i.e. stearic, palmitic, linoleic and/or palmitic acid); after said fatty acids are melted, adding diethylenetriamine (DETA) into the fatty acids in a weight ratio of 1/1 to 1/2 of the fatty acid to diethylenetriamine (DETA); heating the mixture to a predetermined second temperature value (i.e. 120-160°C) in order to perform esterification reactions; heating the fatty acids esterified through the esterification reactions to a predetermined third temperature value (i.e. 180-220°C) and keeping it for a predetermined first time period (i.e 2.5-10 hours) in order to obtain an imidazoline ring structure, cooling the resulting imidazoline ring structure to a predetermined fourth temperature value (i.e. 60-80°C) and adding chloroacetic acid thereto and keeping it for a predetermined second time period (i.e. 2-6 hours) to perform a quaternarization reaction in order to obtain an imidazoline-based corrosion inhibitor.

The step of performing an esterification reaction comprises the step of adding at least one catalyst which is ethyl acetate into the mixture of fatty acid and diethylenetriamine. The ratio of ethyl acetate to fatty acids is preferably 10-50% by weight. In this embodiment, after the catalyst is added into the mixture of fatty acid and diethylenetriamine, water vapor is generated due the reactions performed. Here, the amount of the resulting water vapor is used to determine whether esterification reaction is completed. When the water vapor reaches to a predetermined value, it is understood that esterification reaction is completed Said step of obtaining an imidazoline ring structure is initiated after completion of the esterification reaction, wherein the reactions are performed as follows.

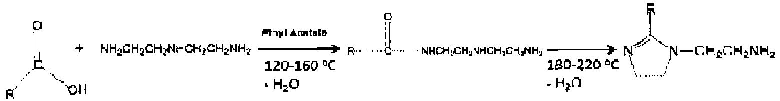

Table 1 shows FT-IR test results of the imidazoline-based corrosion inhibitor obtained in this embodiment and Table 2 shows NMR (Nuclear Magnetic Resonance) results thereof.

**Table 1. FT-IR results of imidazoline-based inhibitor**

| Bond Structure | Wave Number(cm⁻¹) |
|---|---|
| Imidazoline Ring C=N Stretching | 1604 |
| C=O Secondary Amide | 1665 |

**Table 2. NMR results of imidazoline-based inhibitor**

| Bond Structure | ppm |
|---|---|
| CH₃ | 0.81 |
| NH₂ | 1.54 |
| CH₂-NH₂ | 2.15 |
| N-CH₂ | 2.72 |
| CH₂-N | 3.31 |
| CH₂N=C | 3.62 |

In the method of synthesizing a corrosion inhibitor according to the present invention, since waste vegetable oils are used as raw material, production costs are kept low. Furthermore, given that said waste materials are harmful to the environment utilization of such materials in synthesis of a corrosion inhibitor contributes to the protection of the environment.

## Claims

1. A method for synthesizing a corrosion inhibitor, comprising the steps of;
heating the waste vegetable oils to a predetermined first temperature value in order to melt fatty acids contained in the oil; after said fatty acids are melted, adding diethylenetriamine into the fatty acids in a weight ratio of 1/1 to 1/2 of the fatty acid to diethylenetriamine; heating the mixture to a predetermined second temperature value in order to perform esterification reactions; heating the fatty acids esterified through the esterification reactions to a predetermined third temperature value and keeping it for a predetermined first time period in order to obtain an imidazoline ring structure; cooling the resulting imidazoline ring structure to a predetermined fourth temperature value and adding chloroacetic acid thereto; keeping it for a predetermined second time period to perform a quaternarization reaction in order to obtain an imidazoline-based corrosion inhibitor **characterized in that** the esterification reaction comprises the step of adding at least one catalyst which is ethyl acetate into the mixture of fatty acid and diethylenetriamine.

2. A method according to claim 1, **characterized in that** said predetermined first temperature value is in the range of 50-80°C.

3. A method according to claim 1, **characterized in that** said predetermined second temperature value is in the range of 120-160°C.

4. A method according to claim 1, **characterized in that** said predetermined third temperature value is in the range of 180-220°C.

5. A method according to claim 1, **characterized in that** said predetermined fourth temperature value is in the range of 60-80°C.

6. A method according to claim 1, **characterized in that** said predetermined first time period is in the range of 2,5-10 hours.

7. A method according to claim 1, **characterized in that** said predetermined second time period is in the range of 2-6 hours.

8. A method according to claim 1, **characterized in that** the ratio of ethyl acetate to fatty acids is in the range of 10-50% by weight.

## Patentansprüche

1. Verfahren zur Synthese eines Korrosionsinhibitors, mit den Schritten:
Erwärmen der pflanzlichen Altöle auf einen vorgegebenen ersten Temperaturwert, um in dem Öl enthaltene Fettsäuren zum Schmelzen zu bringen, Hinzufügen, nachdem die Fettsäuren geschmolzen sind, von Diethylentriamin zu den Fettsäuren in einem Gewichtsverhältnis von 1/1 bis 1/2 von Fettsäuren zu Diethylentriamin, Erwärmen des Gemisches auf einen vorgegebenen zweiten Temperaturwert, um Veresterungsreaktionen zu bewirken, Erwärmen der durch die Veresterungsreaktionen veresterten Fettsäuren auf einen vorgegebenen dritten Temperaturwert und Halten der Temperatur für eine vorgegebene erste Zeitperiode, um eine Imidazolin-Ringstruktur zu erhalten, Kühlen der resultierenden Imidazolin-Ringstruktur auf einen vierten Temperaturwert und Hinzufügen von Chloressigsäure, Aufbewahren für eine vorgegebene zweite Zeitperiode, um eine Quarternisierungsreaktion zu bewirken, um einen Korrosionsinhibitor auf Imidazolinbasis zu erhalten, **dadurch gekennzeichnet, dass** die Veresterungsreaktion den Schritt beinhaltet, wenigstens einen Katalysator, der Ethylacetat ist, in die Mischung der Fettsäuren und des Diethylentriamins hinzuzufügen.

2. Verfahren nach Anspruch 1, **dadurch gekennzeichnet, dass** der vorgegebene erste Temperaturwert in dem Bereich 50-80°C liegt.

3. Verfahren nach Anspruch 1, **dadurch gekennzeichnet, dass** der vorgegebene zweite Temperaturwert in dem Bereich 120-160°C liegt.

4. Verfahren nach Anspruch 1, **dadurch gekennzeichnet, dass** der vorgegebene dritte Temperaturwert in dem Bereich 180-220°C liegt.

5. Verfahren nach Anspruch 1, **dadurch gekennzeichnet, dass** der vorgegebene vierte Temperaturwert in dem Bereich 60-80°C liegt.

6. Verfahren nach Anspruch 1, **dadurch gekennzeichnet, dass** die vorgegebene erste Zeitperiode in dem Bereich 2,5-10 Stunden liegt.

7. Verfahren nach Anspruch 1, **dadurch gekennzeichnet, dass** die vorgegebene zweite Zeitperiode in dem Bereich 2-6 Stunden liegt.

8. Verfahren nach Anspruch 1, **dadurch gekennzeichnet, dass** das Verhältnis von Ethylacetat zu Fettsäuren in dem Bereich 10-50 Gew.-% liegt.

## Revendications

1. Procédé pour synthétiser un inhibiteur de corrosion, comprenant les étapes suivantes :
le chauffage des huiles végétales usagées jusqu'à une première valeur de température prédéterminée afin de fondre les acides gras contenus dans l'huile ; après que lesdits acides gras sont fondus, l'ajout de diéthylènetriamine aux acides gras à un rapport en poids de 1/1 à 1/2 de l'acide gras sur la diéthylènetriamine ; le chauffage du mélange jusqu'à une deuxième valeur de température prédéterminée afin d'effectuer des réactions d'estérification ; le chauffage des acides gras estérifiés par les réactions d'estérification jusqu'à une troisième valeur de température prédéterminée et son maintien pendant une première période de temps prédéterminée afin d'obtenir une structure cyclique d'imidazoline ; le refroidissement de la structure cyclique d'imidazoline résultante jusqu'à une quatrième valeur de température prédéterminée et l'ajout d'acide chloroacétique à celle-ci ; son maintien pendant une seconde période de temps prédéterminée pour effectuer une réaction de quaternisation afin d'obtenir un inhibiteur de corrosion à base d'imidazoline **caractérisé en ce que** la réaction d'estérification comprend l'étape d'ajout d'au moins un catalyseur qui est l'acétate d'éthyle au mélange d'acide gras et de diéthylènetriamine.

2. Procédé selon la revendication 1, **caractérisé en ce que** ladite première valeur de température prédéterminée est dans la plage allant de 50 à 80 °C.

3. Procédé selon la revendication 1, **caractérisé en ce que** ladite deuxième valeur de température prédéterminée est dans la plage allant de 120 à 160 °C.

4. Procédé selon la revendication 1, **caractérisé en ce que** ladite troisième valeur de température prédéterminée est dans la plage allant de 180 à 220 °C.

5. Procédé selon la revendication 1, **caractérisé en ce que** ladite quatrième valeur de température prédéterminée est dans la plage allant de 60 à 80 °C.

6. Procédé selon la revendication 1, **caractérisé en ce que** ladite première période de temps prédéterminée est dans la plage allant de 2,5 à 10 heures.

7. Procédé selon la revendication 1, **caractérisé en ce que** ladite seconde période de temps prédéterminée est dans la plage allant de 2 à 6 heures.

8. Procédé selon la revendication 1, **caractérisé en ce que** le rapport entre l'acétate d'éthyle et les acides gras est dans la plage allant de 10 à 50 % en poids.
